# EUROPEAN PATENT APPLICATION

(11) **EP 2 218 401 A1**
(43) Date of publication of application: **18.08.2010**
(21) Application number: 09152941.2
(22) Date of filing: 16.02.2009
(51) Int. Cl.: A61B 5/22

(54) **Dexterity device**

(71) Applicant: Valero-Cuevas, Francisco, La Crescenta CA 91214 (US); Forssberg, Hans, 182 39 Danderyd (SE)
(72) Inventor: Valero-Cuevas, Francisco, La Crescenta CA 91214 (US); Forssberg, Hans, 182 39 Danderyd (SE)
(74) Representative: Holmberg, Nils Anders Patrik

(57) **Abstract**

Dexterity device for monitoring, diagnosing, and training a person's dexterity, comprising first and second end parts connected together by a compression means. During use the device is held between one or many fingers being in contact with the end parts, and the end parts are then pressed towards each other resulting in a compression of the compression means. The device further comprises sensor means arranged at one or both end parts at the surfaces intended to be in contact with the fingers during use of the device. The sensor means is adapted to sense and measure the forces applied to the end parts and/or the motions of the end parts, wherein the sensor means is adapted to generate sensor signals in dependence of measured forces and/or motions. The sensor signals are applied to a processing unit for monitoring, diagnosing or training purposes based upon said sensor signals.

## Description

### Field of the invention

The present invention relates to a device according to the preamble of the independent claim.
This invention relates generally to devices for developing and measuring grasping force and grasping dexterity. In particular, it relates to compressible devices that are compressed by a person's digits to develop and measure the person's grasping force and grasping dexterity. The device can be used in a regiment that allows for quantification, measurement and development of motor skills needed for grasping.

### Background of the invention

Adequate grasping force and grasping dexterity is required for individuals to perform tasks like eating, tying shoe laces and thousands of other everyday tasks. When an individual looses grasping strength or control, their independence and quality of life is severely compromised. Loss of grasping strength and dexterity can result from old age and various other health related causes such as injury or disease. The loss of grasping strength and dexterity may be temporary, as is often the case after a person experiences an injury or orthopaedic surgery and rehabilitation. The speed, at which a person can regain proper operation of the hands and digit control after surgery or disease, greatly depends on the amount and quality of physical therapy that the individual receives. Personal physical therapists can be very expensive and are not available to each and every patient that requires therapy to regain grasping strength and dexterity.

Thus, patients that cannot afford physical therapy or do not have such services available for one reason or another have to rely on self motivation in order to develop or regain proper operation of the hands after injury. Unfortunately, if the patient is bed ridden he or she will not have the opportunity to tie shoes, wash dishes and the like which can help them to regain dexterity and strength in the hands.

There are several other reasons why an individual may wish to improve his or her grasping strength or grasping dexterity. For example, musicians that use their hands to play instruments may wish to exercise their fingers in environments where practicing their instrument is not feasible or not possible. Rock climbers may wish to improve their grasping strength prior to a climb and surgeons may wish to improve their dexterity to improve their ability to perform delicate operations.

Physical therapy requires the measurement and development of different combinations of finger strength and dexterity.

A device for developing and measuring grasping force and grasping dexterity is disclosed in US-6,537,075 by one of the inventors to the present application. This known device achieves some of the objects referred to herein. However, it has been observed that it is sometimes a lengthy procedure to perform a complete dexterity monitoring when using the device disclosed in US-6,537,075, inter alia due to the fact that a large number of tests must be performed using a set of test devices. The number of test devices may be as many as 50-70. Furthermore, as the tests must be manually supervised and the results input into a protocol some inaccuracies might occur due to subjective monitoring by the supervisor, and, in addition, the lengthy procedure makes the test rather expensive to perform.
In US 6,537,075 are described different devices with springs, which seem to be intended to be fully compressed in the tests. A series of springs with different compressibility have been used in test programs. The patent briefly mentions a great number of various embodiments, including monitoring (counting) systems connected to the device for monitoring (un)successful compression. As an example, an electrical counter is attached to an external meter.

Therefore, there is a need for an improved and more user-friendly device that can be used to monitor, diagnose, and train the grasping strength and grasping dexterity, and the general object of the present invention is to achieve such a device.

One specific object of the present invention is thus to provide a more user-friendly hand-held device that may be used in a therapy to exercise the digits of an individual in order to improve finger strength. The hand-held device allows individuals to remain substantially immobilized while exercising and developing their grasping strength.

It is a further object of the present invention to provide a device, which is flexible and measures grasping dexterity. The device not only allows the individual to develop grasping strength, but also his or her grasping dexterity.

It is yet another object of the current invention to provide a device for developing grasping strength and grasping dexterity, which is inexpensive, can reduce the cost of therapy, and allows individuals to develop grasping strength and grasping dexterity in a variety of environments.

Still another object of the present invention is to obtain continuous, more high-resolution measures of compression, and to be able to investigate the time history of the compression.

It is also an object of the present invention to provide a device for developing grasping strength and grasping dexterity that yields an objective and quantitative measurement of grasping strength and grasping dexterity.

### Summary of the invention

The above-mentioned objects are achieved by the present invention according to the independent claim.

Preferred embodiments are set forth in the dependent claims.

The device comprises a compression means, which may be a spring, that a person grasps with one, two, three or more fingers and manipulates. The task of the subject is to compress the spring to its solid length without the spring buckling. The device is provided with a sensor means, e.g. strain gauges, at the contact surfaces to sense finger motions and forces. The device will be coupled on line to a processor unit, e.g. a computer, which by measuring e.g. the vector of the finger tip forces will be able to assess dexterity in an objective and reliable manner.

In an embodiment of the invention the signals from the sensor means can be used as input signals to computer software for entertainment, training, evaluation, diagnostic or rehabilitation purposes. The task of manipulating the device can be challenging to the sensorimotor system because it requires the forces and motions of the fingers to be appropriately regulated to respond to each other, the behaviour of the device, or any other external and internal perturbations.

In another embodiment the device is arranged to require different levels of force or that it will become unstable as one manipulates it, thus presenting a variety of levels of difficulty in strength and dexterity parameters, respectively. The information that is generated during manipulation is a rich and time-varying set of signals that offer a direct measurement of the interaction between the device (object and computer) and the brain (sensorimotor system) of the person.

The time history of the signals thus allows the user to provide a rich, context-dependent and dynamic input to the computer to, for example, control software or systems. The time history of the recorded signals also contains information to reconstruct the sensory and motor interactions to accomplish the task, or to identify why the task failed. Engineering analysis can then be used to propose mechanisms that explain these recorded signals, and in so doing, describe the functional features of the sensorimotor circuitry of the user, their skill level. By supplementary research by functional brain imaging, the anatomical localisation of the brain circuits involved in the sensorimotor control can be identified. The dexterity device will therefore be able to reveal impairments of the brain in an objective and quantitative manner, as well as be used as a training tool improving impaired hand motor function in a variety of neurological disorders. The training paradigms can be developed to produce specific changes in the brain circuits and peripheral nervous systems, musculature and skeletal tissues of the user to enhance manipulation ability.

The present invention can be used to objectively monitor fine hand motor control in a quantitative manner. Hand/finger function (dexterity) is impaired in several neurological disorders in children (CP, DCD, ADHD) and adults (stroke, brain injuries, aging). Today there are no good objective and quantitative assessments tools for dexterity, while there is a great need for such simple clinical tools. In addition, the dexterity device according to the present invention can be coupled to a PC to control computer games which can be constructed in order to train fine motor skills dexterity. Thus, the device consists of compression means that should be compressed between the thumb and the index finger, and sensor means measuring the finger tip forces. By setting specific mechanical requirements of strength and dexterity, the device allows complex interactions between the brain, the hand and the physical world.

This device solves the problem of dynamically extracting multiple commands or degrees of control from the nervous system, as when controlling software and hardware. It also solves the problem of measuring, testing, and assessing the ability of the brain-and-hand to perform tasks of varying complexity and difficulty. It also solves the problem of training and teaching people to perform tasks of varying difficulty. We are primarily interested in extracting information about the sensory-motor system manifest in grasping and manipulating the object described herein.

This device can also be used as a multiple degree of freedom control input for a computer, game, training protocol, rehabilitation or robotic device, and as a feedback tool in teleoperated robotic devices or other uses such as they may be.

There are many neurological diseases in children, adolescents and adults that affect the motor system and that lead to motor impairments. The most common and applicable for the dexterity test are, cerebral palsy, developmental coordination disorders, stroke, and acquired brain injuries. Hand function is critical for activity of daily living and even small impairments in hand function affect the patient's ability. It is therefore important in the clinical work to have good tools measuring hand function and also to have good methods for training and rehabilitation of hand function. Even a small progress might be important, which means that the instrument also should be able to measure small nuances in performance. The dexterity device is considered to meet both this needs and is, in particular, generally applicable in the following two different lines:
1 Fine motor test for diagnostics and assessment of finger dexterity. It will be an objective method, giving quantitative data with high resolution, i.e. detecting small differences.
2 Training method for hand motor skills. The device will be coupled to a computer and special computer games aiming at training finger dexterity will be constructed. The training will be home based, and by internet coupling each training session will be monitored and send to the responsible therapist who can monitor the progress of the training, and give instructions and adjusting the difficult level of the training.

According to one embodiment the device is arranged for controlling e.g. computer games or the like.

The device according to the present invention consists of a spring which preferably is impossible to fully compress, in contrast to the springs that are described in the above-mentioned prior art which must be fully compressed to achieve an approved test performance.
The forces applied to the training device according to the present invention are detected and monitored by sensors attached to the movable parts of the device, in contrast to prior art in which the performance of test is visually evaluated or measured with respect to ground. The signals from the device will be fed on line to a computer. Special software is available that monitors and stores the "analogue" quantitative value of dexterity for each compression trial, which will be used as a clinical measure of dexterity.
Furthermore, for training and rehabilitation, the device is equipped with sensors sending signals to the computer which will be used for controlling computer games. Special software is designed to train the dexterity by instructing the person to compress and move the device. As an example, a home-based training program may be used, allowing distance communication with the therapist over internet following the training and being able to adjust the programme and the training appropriately. Specific neural circuits can be promoted or enhanced with direct application to the deficits found in populations of all ages like stroke and cerebral palsy.

In summary the present invention is applicable in numerous different fields; some are listed in the following:
A clinical method to monitor manipulation, grasping, fine motor skills and dexterity in subjects with neurological impairments affecting hand motor control.
A research method in clinical studies in order to evaluate various interventions and therapies.
A training device to improve hand motor skills and dexterity by enhancing sensorimotor, cognitive, learning and perceptual functions.
A clinical device to train manipulation ability in clinical populations needing improvement of hand or brain function.
Computer input device for games and active/dynamic/precise control of a computer, game, system or robot, for example.
Devices for simple entertainment, such as toys, games, etc.
An active force-feedback device for the control of engineering systems or software.
A metric for grasping prostheses, robots, or people with grasp degeneration.

### Short description of the appended drawings

Figure 1 is a schematic side view illustration of a dexterity device according to the present invention.
Figure 2 is a schematic illustration of the dexterity device shown in figure 1 in a compressed state.
Figure 3 is a schematic top view illustration of a of a dexterity device according to the present invention.
Figure 4 is a schematic illustration of a set of dexterity devices according to the present invention.
Figure 5 shows a diagram illustrating the relationship of sensed forces and a stability index when applying the dexterity device according to the present invention.
Figure 6 is a block diagram illustrating different applications of the dexterity device.

### Detailed description of preferred embodiments of the invention

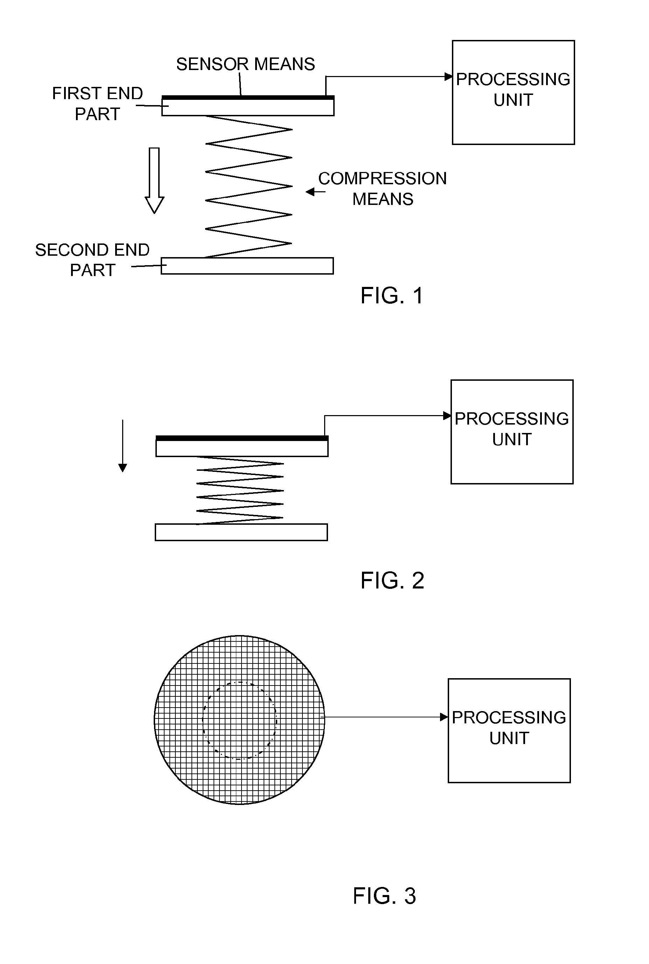

The present invention will now be described in detail with references to the figures.
In a preferred embodiment of the invention the device is configured to be held between one of more fingers and a thumb of the same hand. The user compresses the device in a compression direction, indicated by an arrow in figure 1, with the finger, or fingers, and the thumb by applying the appropriate force along a linear compression direction resulting in a compression, deformation or movement of the compression means. In the most preferred embodiment of the invention, the device is also capable of bending or flexing (buckling) in an off axis direction such that the user is required to consciously compress the device along the linear compression direction with a certain degree of dexterity to achieve successful compression. The ease with which the device is designed to bend in an off axis direction determines the required grasping dexterity that the user must have to successfully compress the device. For this purpose coiled springs are particularly useful.

The device can be configured to accommodate any number of digits.

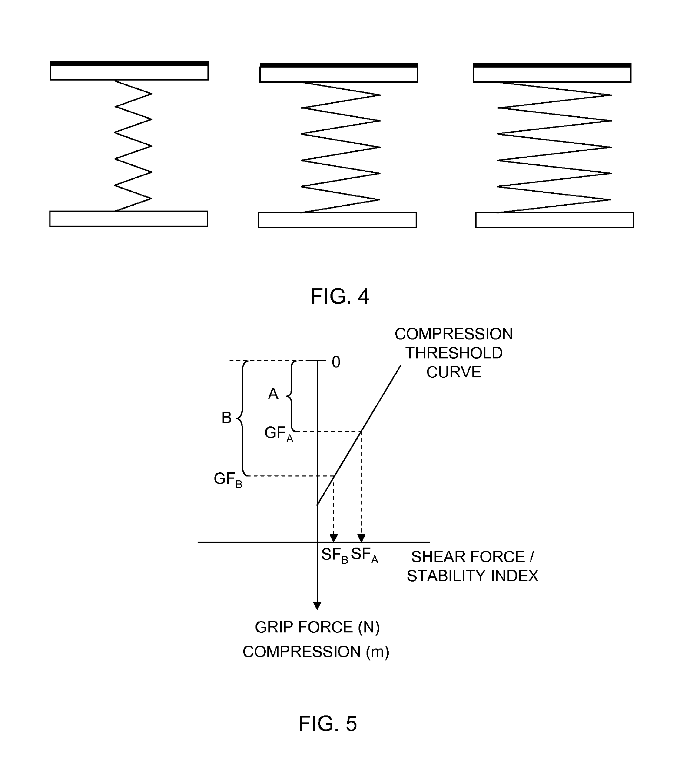

Figure 4 is used to illustrate variations of compression strength and dexterity required to obtain a linear compression of springs having various physical properties. A compliant spring requires less force to be compressed, schematically illustrated by device to the left in figure 4, while a stiff spring requires more force to be compressed, the device to the right. A wide, short spring is more stable because it requires a less precise alignment of forces to compress, while a narrow, long spring is more precarious because it requires a more precise alignment of forces to compress, thus requiring a greater degree of dexterity to compress. Different types of springs can be used to measure different combinations of finger force and grasping dexterity. For example, a lower degree of finger force and grasping dexterity can be measured with a wide (25 mm diameter), short (12 mm length), compliant (0.1 N/mm stiffness) spring, while a higher degree of finger force and grasping dexterity can be measured with a narrow (8 mm diameter), long (50 mm length), stiff (2 N/mm stiffness) spring.

In addition, the dexterity of the patient or trainee's grasping ability can be quantifiably measured by varying the materials used in construction of the end parts placed at the ends of the spring. End parts with high surface friction, such as sand paper or carpet, can be used to test low dexterity. End parts with low surface friction, such as Teflon® or smooth plastic, can be used to test high dexterity. End parts with intermediate surface friction, such as wood or cloth, can be used to test intermediate dexterity. Obviously, the more slippery the material used, the more difficult it will be for the patient or trainee to grasp and align the end parts and then squeeze them together with an opposing force. By varying the texture of the surface, the sensory information of the fingers can be varied and used to quantify and test the influence of finger sensation on grasping ability.

Figure 1 is a side view of an embodiment which can be used to measure and develop grasping force between a thumb and a finger. This embodiment is exemplified by a device which utilizes a compression means, e.g. a spring. The device is provided with a first end part attached to a first end of the compression means and a second end part attached to the opposing end of the compression means. The device is designed for compression along a linear compression direction, indicated by an arrow, by applying an appropriate force to the end parts and is adapted to be used for monitoring, diagnosing, and training a person's dexterity.
According to a preferred embodiment the compressible means is a helical compression spring and the end parts being e.g. in the form of planar discs. Naturally, numerous alternative embodiments regarding the end parts exist as long as they are able to perform their intended use, i.e. to be a support for the fingers/thumb during use of the device. Alternative structures of the end part may be a ring or tube attached to the compressible means such that the finger/thumb may be inserted into the ring or tube upon use.

In alternative embodiments the compressible means of the device may be made from a variety of pliable, flexible or resilient materials including plastic, rubber and foam rubber. Alternatively, the compressible section of the device may be made from a combination of different compressible materials.
The compressible means that have been described above return to its original extended position in the absence of an applied force.

During use, as being discussed above, the device is held between one or many fingers being in contact with the end parts, and the end parts are then pressed towards each other resulting in a compression of the compression means.

The device further comprises sensor means adapted to sense and measure the forces applied to the end parts and/or the motions of the end parts. Furthermore, the sensor means is adapted to generate sensor signals in dependence of the measured forces and/or motions, and the sensor signals are applied to a processing unit for monitoring, diagnosing or training purposes based upon the sensor signals.

According to a preferred embodiment sensor means is arranged at one or both of the surfaces intended to be in contact with the fingers during use of the device in order to sense and measure the forces applied to the one or both end part(s).

As an alternative, in the case the movement of the end plates in relation to each other is to be measured the sensor means is an acceleration measuring means adapted to measure the acceleration and/or the movement of the end plates in relation to each other. In this embodiment the sensor means needs not to be in contact with the fingers in order measure the movement/acceleration.
Similarly, the acceleration and/or movement of the end plates in relation to each other may also be detected by optical means, or by an electromagnetic sensor, where e.g. a small magnet may be arranged in connection with one end plate and e.g. a coil to detected the magnetic field is arranged in connection with the other end plate to detect motion of the fingers and device.

According to one embodiment the sensor means is a Force Sensing Resistor (FSR) sensor. An FSR-sensor is a polymer thick film (PTF) device which exhibits a decrease in resistance with an increase in the force applied to the active surface. Its force sensitivity is optimized for use in human touch control of electronic devices. FSRs are not a load cell or strain gauge, though they have similar properties. The FSR sensing means may be arranged in many different circuitries in dependence of the chosen application.

In a basic circuitry the sensor means comprising the FSR sensor, a measuring resistor (RM) and an operational amplifier. The output voltage of the amplifier increases with increasing force applied at the FSR. The measuring resistor, RM, is chosen to maximize the desired force sensitivity range and to limit current. The current through the FSR should preferably be limited to less than 1 mA/square cm of applied force.

According to another embodiment the sensor means is a light thin force (LTF) sensor. Examples of such existing sensors are force sensing resistors, thin-film capacitive sensors, miniature load cells with strain gauges, miniature laser diffraction sensors which exhibits a change in electrical, electromagnetic or optical signal with a change in the force applied to the active surface. These types of sensors may sense only the force perpendicular to the surface, or all three components of force. In addition, there could also be no force sensors but rather position sensors that measure the relative motion of the ends of the spring or the fingers with high accuracy. Such systems include cameras that triangulate the position of markers on the springs or hand, or use electromagnetic sensors, etc. Currently, for the sake of example, the preferred embodiment uses commercially available force sensing resistors and miniature load cells.

Thus, according to other embodiments the sensor means being a strain gauge, or button load cell, or any other form of thin-membrane sensor.

And in still another embodiment the sensor means being a piezo-electric sensor.

According to still other embodiments the sensors would be arranged on or embedded in the helical compression spring, or in the material that comprises the compression means.

In order to achieve high accuracy measurements the sensor means, in accordance with an alternative embodiment, is a sensor array having a two-dimensional extension and comprises a number of sensor cells, where each sensor cell measures the force acting on it.

The processing unit is connected to the sensor means via an electrical cable used to supply energy to the sensor means and to transmit sensor signals to the unit.

In an alternative embodiment the sensor signals are wirelessly transferred to the processing unit, using e.g. Bluetooth or any other available wireless protocol. In that case the sensor means must be energized via a battery or similar e.g. arrange within the end plates.
Also, techniques like induction coupling or the like may be used to power it in a wireless way.
Furthermore, the sensor means may be so-called passive sensors where the forces or movements suffice to elicit the sensor signals.

The sensor signals obtained by the sensor means are applied to the processing unit, either as raw, non-processed signals, or processed and amplified signals. If the sensor signals need to be further processed the processing unit is provided with applicable circuitry to achieve that processing.
The signals from the device will be fed on line to the processing unit, e.g. a personal computer. Special software is used to monitor and store the "analogue" quantitative value of dexterity for each compression trial, which will be used as a clinical measure of dexterity.

Figure 5 shows a diagram illustrating the relationship of sensed forces and a stability index when applying the dexterity device. In one embodiment the sensor signals represent the grip forces measured by the sensor means. Prior the device is compressed the sensed force is 0, or close to 0, indicated by the dotted horizontal line in the figure. As the device is compressed the sensed force increases in dependence of the compressed distance, in the diagram the downward direction represents an increasing force. In one type of measurements, the device is compressed until it buckles, the measured force at that moment is detected and a corresponding shear force, or stability index, is determined as illustrated in the diagram. This is achieved by applying a compression threshold curve that characterizes the used dexterity device. The compression threshold curve is determined in dependence of inter alia the resiliency of the compression means.

In the figure is illustrated two different measurements. In one measurement the user manages to compress the device a distance A, by using a maximal grip force of GF_{A} resulting in a corresponding SF_{A}.
In the other measurement the compressed distance B results in a shear force (stability index) of SF_{B}.

In order to determine if the dexterity test performed by a user during a measurement session resulted in a successful or non-successful compression a set of dexterity criteria is available.

According to one embodiment the dexterity criterion relates to the vector of forces applied during a measurement test. The compression means has a defined compression direction being the direction the dexterity device is to be compressed during use of the device which in turn defines an optimal vector direction. More specifically, the finger tip forces are determined and the processing unit is adapted to assess the dexterity based upon the determined vector and a successful compression is achieved if the determined vector does not deviate more than a predetermined angle from the compression direction. The angle is chosen in dependence of many circumstances, e.g. the resiliency of the compression means and an exemplary angle is typically in the range of 5-45 degrees, preferably 10-20 degrees, e.g. 15 degrees.

According to another embodiment a dexterity criterion is used based upon the compression threshold curve illustrated and explained in relation to figure 5.

According to still another embodiment a dexterity criterion based upon differences between the determined movement pattern of the end parts and stored dexterity templates representing specific dexterity situations.

In order to meet different user's needs often a set of dexterity devices, e.g. 2-4, is used, where each dexterity device has a unique set-up, e.g. with regard to required compression force for a given compression length, size of the end parts, etc.

In order to perform a successful trial a number of different alternatives exists. Below is discussed two different alternatives:
1. The user compresses the compression means as much as possible without loosing the grip and without buckling the compression means, and then releases the pressure such that the compression means expands and returns to the initial uncompressed state. A presumption for a successful trial is that the whole procedure is performed without loosing the grip and without buckling.
2. The user compresses the compression means without buckling and keeps the device compressed for a predetermined time period, e.g. in the interval of 2-25 seconds, preferably 3 seconds or in another application 20 seconds, and then, in a controlled fashion, releases the pressure allowing the compression means to expand and to return to the initial state. As in the first alternative, a presumption for a successful trial is that the whole procedure is performed without loosing the grip and without buckling.

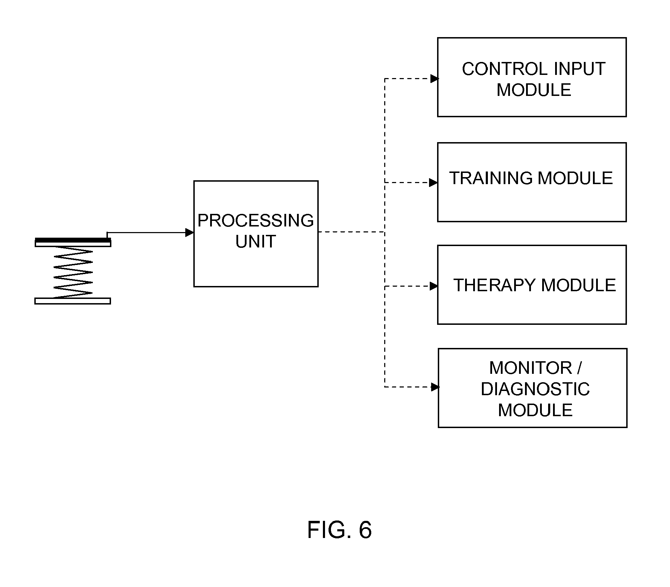

Figure 6 is a block diagram illustrating different applications of the dexterity device, each module may be used alone or in combination with one or many modules.

In one embodiment the sensor signals are applied to a monitor module adapted to monitor manipulation, grasping, fine motor skills and dexterity in particular in subjects with neurological impairments affecting hand motor control.

In another embodiment the sensor signals are applied to a therapy module adapted to evaluate various interventions and therapies.

In still another embodiment the sensor signals are applied to a training module to improve a patent's hand motor skills and dexterity by enhancing sensorimotor, cognitive, learning and perceptual functions.

In a further embodiment the sensor signals are applied to a control input module for games and active/dynamic/precise control of e.g. a computer, a game, a system or a robot, or applied to a control input device to any system to be controlled or manipulated, or explored, and where it functions as an entertainment, recreational or training device/activity.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Dexterity device for monitoring, diagnosing, and training a person's dexterity, the device comprises first and second end parts connected together by a compression means,
during use the device is held between one or many fingers being in contact with the end parts, and the end parts are then pressed towards each other resulting in a compression, deformation or movement of the compression means,
**characterized in that** the device further comprises sensor means adapted to sense and measure the forces applied to the end parts and/or the motions of the end parts, wherein the sensor means is adapted to generate sensor signals in dependence of measured forces and/or motions, said sensor signals are applied to a processing unit for monitoring, diagnosing or training purposes based upon said sensor signals.

2. Dexterity device according to claim 1, wherein said sensor means is arranged at one or more of the surfaces intended to be in contact with the fingers during use of the device in order to sense and measure the forces applied to the one or both end part(s).

3. Dexterity device according to claim 1, wherein if the sensor signals fulfil predefined dexterity criteria during a measurement session the dexterity test performed during the session is classified as a successful compression.

4. Dexterity device according to claim 1, wherein the processing unit is adapted to determine, from said sensor signals, the vector of the finger tip forces and to assess the dexterity based upon said determined vector.

5. Dexterity device according to claim 4, wherein the compression means has a defined compression direction being the direction the dexterity device is to be compressed during use of the device.

6. Dexterity device according to claim 5, wherein a successful compression is achieved if the determined vector does not deviate more than a predetermined angle from the compression direction, said angle is in the range of 10-20 degrees, preferably 15 degrees.

7. Dexterity device according to any of preceding claim, wherein the sensor signals obtained during a specified time period define a measurement session, said specified time period is in the range of 1-4 seconds.

8. Dexterity device according to any preceding claim, wherein said compression means being a helical compression spring and said end parts being in the form of planar discs.

9. Dexterity device according to claim 1, wherein the sensor means is a sensor array having a two-dimensional extension and comprises a number of sensor cells, where each sensor cell measures the force acting on it.

10. Dexterity device according to any preceding claim, wherein said sensor means is a Force Sensing Resistor (FSR) sensor.

11. Dexterity device according to any of claims 1-9, wherein said sensor means being a strain gauge.

12. Dexterity device according to any of claims 1-9, wherein said sensor means being a piezo-electric sensor.

13. Dexterity device according to any of claims 1-9, wherein said sensor means is an acceleration measuring means adapted to measure the acceleration and/or the movement of the end plates in relation to each other.

14. Dexterity device according to any preceding claim, wherein said sensor signals are applied to a monitor module adapted to monitor manipulation, grasping, fine motor skills and dexterity in particular in subjects with neurological or orthopedic impairments or injury or developmental change or aging or training affecting hand motor control.

15. Dexterity device according to any of claims 1-13, wherein said sensor signals are applied to a therapy module adapted to evaluate various interventions and therapies.

16. Dexterity device according to any of claims 1-13, wherein said sensor signals are applied to a training module to improve a patent's hand motor skills and dexterity by enhancing sensorimotor, cognitive, learning and perceptual functions.

17. Dexterity device according to any of claims 1-13, wherein said sensor signals are applied to a control input module for games and active/dynamic/precise control of e.g. a computer, a game, a system or a robot, or applied to a control input device to any system to be controlled or manipulated, or explored, and where it functions as an entertainment, recreational or training device/activity.

18. A set of dexterity devices according to any preceding claim, wherein each dexterity device has a unique required compression force for a given compression length.
